# EUROPEAN PATENT APPLICATION

(11) **EP 2 039 360 A1**
(43) Date of publication of application: **25.03.2009**
(21) Application number: 07116725.8
(22) Date of filing: 19.09.2007
(51) Int. Cl.: A61K 31/485, A61K 31/4468, A61K 31/135, A61P 25/04

(54) **Non-invasive direct delivery of opioids to the central nervous system**

(71) Applicant: EURO-CELTIQUE S.A., 1653 Luxembourg (LU)
(72) Inventor: Fleischer, Wolfgang, 55218, Ingelheim (DE)
(74) Representative: Bühler, Dirk

(57) **Abstract**

The present invention relates to opioid formulations for treating pain.

## Description

### Field of the Invention

The present invention concerns formulations and methods for the non-invasive direct delivery of opioids to the central nervous system (CNS). More particular, the invention concerns formulations comprising particulate carrier opioid compositions for the application on the skin/mucous membrane of a mammal and subsequent delivery of opioids directly to the central nervous system (CNS).

### Background of the invention

Only limited routes are currently available for delivering substances to the CNS. These routes either deliver the substance directly and invasively to the CNS (direct route) via e.g. an injection into the brain or the cerebrospinal fluid (CSF), or indirectly via systemic distribution and passage across the blood-brain and/or blood-cerebrospinal fluid barriers (systemic route). Systemic routes include direct injection into the blood stream and absorption through the gastrointestinal tract, skin and mucous membranes. Systemic distribution suffers from difficulties in crossing the blood-brain and/or blood-CSF barriers and from adsorption of the substances by other parts of the body, reducing the accuracy of dosing and the overall efficacy, and often causing adverse side effects. Generally, routes using the gastrointestinal tract are even less preferred due to the fact that the substances are metabolized by the enzymes of the gastric, intestinal or rectal mucosa, the intestinal flora, or the liver (first-pass effect), all these involving a reduction in bioavailability and overall efficacy. However, oral application is, despite these drawbacks, widely used due to its ease of application and convenience. For patients needing constant medication, e.g. patients suffering chronic severe pain, ease of application and convenience is of major importance.

Speaking generally, direct application of the drug at the receptor site is advantageous with respect to accurate dosing, efficacy and reduced adverse side effects. When the CNS includes the target receptor site, direct routes include the epidural and intrathekal routes of application. For an epidural application, the drug is injected in the epidural space located immediately outside of the protective sac which contains the spinal cord, whereas for an intrathekal application the drug is injected into the protective sac and into the CSF. It is apparent, that despite of the advantages due to the direct application, issues of safety, ease of use and comfort permit only limited use of this method.

As stated above, patients with chronic severe pain need constant medication. Safety, ease of use and comfort are crucial for life quality and therefore restrict the usefulness of epidural or intrathekal applications. Especially the emplacement of catheters involves the likelihood of infections and discomfort. Therefore, the majority of patients suffering chronic pain prefer oral application, despite the drawbacks of decreased bioavailability and adverse side effects due to passage of the intestinal tract and the systemic distribution of opioids.

Some improvements have been obtained regarding the epidural route of application.
For example, US 5 931 809 discloses a sustained release drug delivery system for epidural delivery of opioids. The compositions are injected in the epidural space and release the drug from the lipid carrier system over a longer period of time as compared to epidural administration of the same drug in free (immediate release) form. The use of sustained-release formulations simplifies and reduces the overall cost of epidural analgesia by elimination of the need for continuous infusion, multiple bolus injections or emplacement of catheters, and also decreases the likelihood of infections. However, the compositions still need to be injected and therefore safety issues and patient compliance issues are still not optimally addressed.

There is thus still a need for compositions and methods for the direct delivery of opioids to the central nervous system (CNS), avoiding the disadvantages of invasive applications, such as the epidural or intrathekal application routes.

Liposomes are generally known drug or compound carriers and thus the application of medicaments in liposomal form has been the subject of investigation for quite some time. An overview concerning the administration of compounds in liposomal form to the skin is provided by the review "Targeted delivery to the pilosebaceous unit via liposomes" (Lauer, A.C. et al. (1996), Advanced Drug Delivery Reviews, 18, 311-324). This review describes the physical-chemical characterisation of liposomal preparations and their therapeutic applications for the treatment of the pilosebaceous unit. Compounds that have been investigated for delivery by liposomes include e.g. anti-cancer agents, peptides, enzymes, anti-asthmatic and anti-allergic compounds and antibiotics.

Liposomal opioid compositions are known for injection into the CSF or the blood system. These liposomal compositions prolong the release of the opioid and thereby reduce the application frequency. The route of application is again invasive.

For example, the above mentioned document, US 5 931 809, discloses controlled release liposomal opioid composition for epidural injection. These compositions and respective method of application are invasive and although the application frequency is reduced, suffer still from safety and compliance issues. US 0 300 806 discloses release controlled injectable liposomal opioid compositions. The compositions are injected into the blood system. The composition and respective method of application still suffer from the drawbacks of the systemic route of application as well as from safety and compliance issues.

### Objective and summary of the invention

The objective of the invention is to provide formulations for the direct delivery of actives, in particular opioids to the CNS avoiding invasive steps. The object of the invention is further to treat mammals, preferably humans with actives such as opioids via direct non invasive delivery of actives such as opioids to the CNS.

The invention is directed to formulations comprising particulate carrier active compositions (such as a particulate carrier opioid composition). The invention is further directed to the use of the inventive formulations and in particular the particulate carrier active- e.g. opioid-compositions for the manufacture of a medicament for the treatment of mammals, preferably humans with actives such as opioids via direct non invasive delivery of actives such as opioids to the CNS.

The invention is further directed to a method of treating pain in mammals, preferably humans, comprising the treatment with the inventive formulations comprising particulate carrier active- e.g. opioid-compositions thereby achieving direct non invasive delivery of actives such as opioids to the CNS.

The inventive formulations comprise particulate carrier active- e.g. opioid-compositions which comprise at least one active such as an opioid substantially enclosed in a particulate carrier composition wherein said carrier composition is capable of delivering the at least one active such as an opioid to cerebral nerves (cranial nerves) or the spinal cord upon application of said formulations comprising said particulate carrier active- e.g. opioid-compositions to the mammals surface skin or mucous membrane, at the site of said cerebral nerves or the spinal cord, across the respective cell membrane and/or cutaneous barriers between said cerebral nerves or the spinal cord and the external surface of said skin, or mucous membrane.

Particularly suitable for the non-invasive direct delivery of actives such as opioids to the CNS is the delivery via the olfactory nerve. The olfactory nerve is the first of twelve cranial nerves. The olfactory nerve is the shortest of all the twelve cranial nerves and one of the only two cranial nerves (the other being the optic nerve which is also a suitable target for the present invention) that do not join with the brainstem. The olfactory nerve consists of a collection of sensory nerve fibers that extend down from the olfactory bulb and pass through the many openings of the cribriform plate, a sieve-like structure. The specialized olfactory receptor neurons of the olfactory nerve are located in the olfactory mucosa of the upper parts of the nasal cavity.

The olfactory mucosa is an organ made up of the olfactory epithelium and the mucosa, or mucus secreting glands, behind the epithelium. The mucus protects the olfactory epithelium and allows odors to dissolve so that they can be detected by olfactory receptor neurons. In mammals, the olfactory mucosa is located on the roof of the nasal cavity above and behind the nostrils.

In vertebrates, the olfactory epithelium consists of three basic cell types: olfactory receptor neurons; sustentacular cells, a type of supporting cell; and basal cells, the stem cells that continuously give rise to new olfactory receptor neurons and sustentacular cells.

An olfactory receptor neuron, also called an olfactory sensory neuron, is the primary transduction cell for olfaction in the olfactory system. Humans have about 40 mullion olfactory receptor neurons. The cells are bipolar neurons with a dendrite facing the interior space of the nasal cavity and an axon that travels along the olfactory nerve to the olfactory bulb.

Many tiny hair-like cilia protrude from the olfactory receptor cell's dendrite into the mucus covering the surface of the olfactory epithelium. In this case the particular carrier composition can be delivered directly to the nerve substantially without passing any cutaneous barriers.

The inventive particulate carrier compositions directly deliver actives such as opioids to the central nervous system (CNS) across cutaneous barriers into cerebral nerves as e.g. the olfactory nerve or the spinal cord. The opioids are thereby delivered to the CNS substantially avoiding the systemic distribution of the opioids throughout the body. The application is non-invasive and therefore safe and easy to use. The application also substantially avoids systemic distribution of the opioid(s), and therefore allows accurate and minimal dosing and reduces adverse side effects.

The present invention is also directed to any treatment of mammals, preferably humans, by delivering opioids to the CNS via celebral nerves or the spinal cord, in particular via celebral nerves such as the olefactory nerve. The invention is particularly useful for treatments that require constant medication with opioids that are predominantly supposed to react with receptors at the site of the CNS, e.g. the treatment of chronic severe pain.

The present invention is further directed to the manufacture of medicaments for the direct delivery to the cerebral nerves or the spinal cord, in particular the cerebral nerves such as the olfactory nerve.

### Description of the drawings

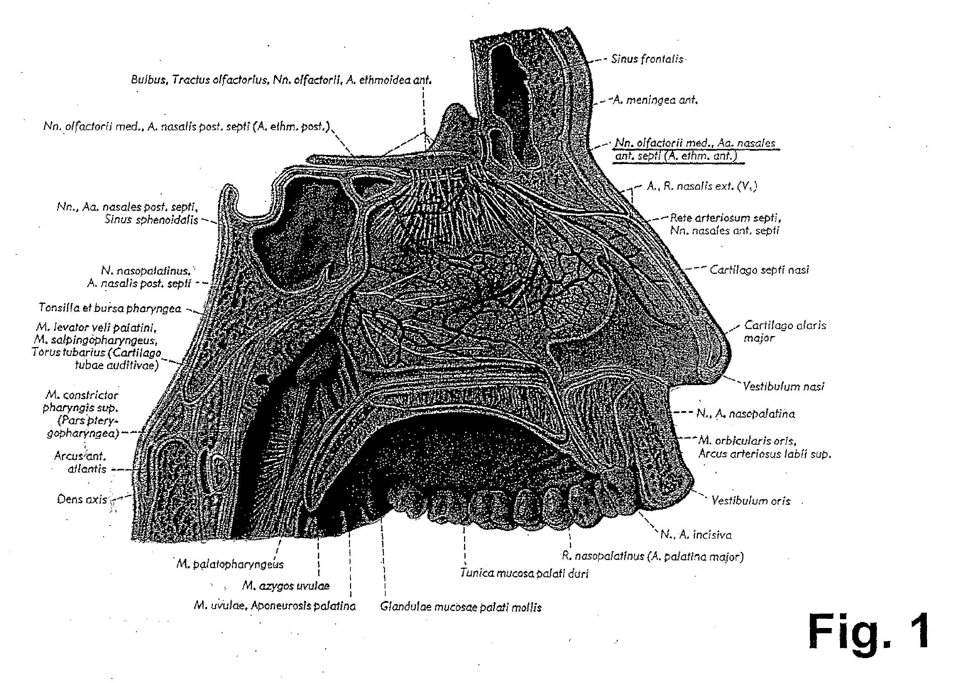

- Fig. 1: shows a cross section of the human head, in particular the area of the nose

### Detailed description of the invention

In the context of the invention "direct delivery" means delivery of substances to the CNS substantially avoiding a systemic distribution of the substance prior to delivery to the CNS, or the cerebral nerves e.g. epidural or intrathekal application or the non-invasive application according to the invention.

In the context of the invention "non-invasive" means without opening a pathway for delivery through a membrane such as skin, by use of apparative means, e.g. by use of injection or the like.

In the context of the invention "particulate carrier composition" includes any particulate carrier composition suitable to facilitate the transport of substances e.g. across cutaneous barriers. The preferred particulate carriers are liposomes.

In the context of the invention "particulate carrier opioid compositions" include any particulate carrier composition suitable to transport substances across cutaneous barriers comprising at least one opioid. The preferred particulate carriers are liposomes.

In the context of the invention, the term "opioid" is considered to include opioid agonists and opioid antagonists, mixed opioid antagonists/agonists as well as mixtures thereof. The term "opioid" includes opioids in the form of the free base as well as pharmaceutically acceptable salts thereof.

In the context of the invention the term "target nerve tissue" and "target nerve" are used interchangeable and include nerve tissue that may transport the opioid(s) to the CNS along its nerve pathway. Preferred "target nerve tissues" are nerves that run close to the body surface, Suitable nerves are cerebral nerves such as the nervus olefactorius, nervus opticus and nervus acusticus. Preferred is the nervus olefactorius.

In the context of the invention the term "cutaneous barriers" include the skin, parts of the skin, membranes, tissue and mucous membranes of a mammal, preferably a human. These cutaneous barriers are between the target nerve tissue and the surface of the skin or mucous membrane of the mammal the inventive compositions are applied to.

In the context of the invention "surface skin or mucous membrane" means those skins or mucous membranes of a mamma! that form the body surface of the mammal and arc exposed to outer environment. Preferably the distance between the target nerve tissue and the surface of the skin or mucous membrane is as short as possible.

In the context of the invention the term "surface skin or mucous membrane at the site of the target nerve tissue (e.g. cerebral nerves) " means the surface skin or mucous membrane closest to the target nerve tissue. Preferred is the olefactory mucosa.

### Opioids/actives

According to one embodiment, the inventive particulate carrier active compositions comprise at least one opioid.

According to the invention, opioid agonists comprise all compounds that belong to class NO2A of opioid analgesics according to the ATC Classification of the WHO. Preferably, an opioid agonist is selected from the group of morphine, oxycodone, hydromorphone, oxymorphone, buprenorphine, fentanyl, afentanyl, sulfentanyl, propoxyphene, nicomorphine, dihydrocodeine, diamorphine, papaveretum, codeine, ethylmorphine, phenylpiperidine and derivates thereof, methadone, dextropropoxyphene, pentazocine, tilidine, tramadol and hydrocodone. Further examples for useable analgesic according to the invention are meperidine, alphaprodine, anileridine, dextromoramide, metopone, levorphanol, phenazocine, etoheptazine, propiram, profadol, phenampromide, thiambuten, pholcodeine, codeine, dihydrocodeinon, 3-trans-dimethylamino-4-phenyl-4-trans-carbethoxy-A'-cyclohexen, 3-dimethylamino-0-(4-methoxyphenyl-carbamoyl)-propiophenone oxime, (-)β-2'-hydroxy-2, 9-dimethyl-5-phenyl-6, 7-benzomorphane, (-)2'-hydroxy-2-(3-methyl-2-butenyl)-9-methyl-5-phenyl-6, 7-benzomorphane, pirinitramide, (-)α-5,9-diethyl-2' hydroxy-2-methyl-6, 7-benzomorphane, ethyl 1-(2-dimethylaminoethyl)-4,5,6,7-tetrahydro-3-methyl-4-oxo-6-phenyl-indol-2-carboxylate, 1-benzoylmethyl-2, 3-dimethyl-3- (m-hydroxy-phenyl) - piperidine, N-allyl-7α (1-R-hydroxy-1-methylbutyl)-6,14-endo-ethanotetrahydronororipavine, (-)2'-hydroxy-2-methyl-6,7-benzomorphane, noracylmethadol, phenoperidine, α-d1-methadol, α -1-methadol, β-d1-acetylmethadol, α-1-acetytmethadol and β-1-acetylmethadol or the pharmaceutically acceptable salts thereof. These lists are not to be understood as exclusive.

Especially preferred analgesically effective opiod agonists are, hydromorphone, morphine, buprenorphin, fentanyl, afentanyl and sulfentanyl, levomethadone, oxycodone, piritramideor the pharmaceutically acceptable salts thereof.

According to the invention, opioid antagonists comprise such compounds that counteract opioid agonists. Such compounds can also be found in the ATC Classification of the WHO. According to the invention, compounds are preferred that upon application in accordance with the invention decrease the side effects, the habituation effects and the addictive potential caused by the opioid agonists. Antagonists can comprise among others, naltrexone, naloxone, nalmefene, nalorphine, nalbuphine, naloxoneazinen, methylnaltrexone, ketylcyclazocine, norbinaltorphimine, naltrindol, 6-β-naloxol and 6-β-naltrexol or the pharmaceutically acceptable salts thereof.

Especially preferred antagonists comprise naltrexone, nalmefene and naloxone or the pharmaceutically acceptable salts thereof. Most preferred is naloxone or a pharmaceutically acceptable salt thereof.

The inventive particulate carrier opioid compositions comprise at least one opioid, e.g. an opioid agonist or an opioid antagonist. The inventive particulate carrier opioid compositions may also comprise the combination of an opioid agonists and an opioid antagonist to reduce side effects and the risk of abuse.

The absolute amounts of agonist and/or antagonist to be used depend on the intended treatment.

In a further embodiment, the opioid agonist may be combined with one or more actives, in particular selected from the group of antidepressants. Suitable antidepressants are moclobemide, tranylcypromine, amitriptyline, amitriptylinoxide, clomipramine, doxepine, maprotiline, lofepramine, trimipramine, dosulepine, imipramine, opiramol, mianserine, nortriptyline, dibenzepine, desipramine, mirtazapine, fluoxetine, fluroxamine, citalopram, paroxetine, sertraline, tryptophane, reboxetine, ulpirid, nefazodone, tranzodone, venlafaxine, viloxazine or the pharmaceutically acceptable salts thereof.

In a further embodiment, the opioid may be combined with any active suitable for delivery to the CNS.

In an even further embodiment, the opioid may be substituted by any other active suitable for delivery to the CNS.

The afore-mentioned active agents may also be used in the form of their pharmaceutically acceptable salts. Preferred salts may be acid addition salts such as the hydrochloride salt. Typical examples are oxycodone hydrochloride and naloxone hydrochloride.

### Particulate carriers

The inventive formulations comprise at least one particulate carrier composition. Preferred particulate carriers are liposomes.

The known prior art methods for forming liposome structures can generally be used in the context of the invention. Broadly, these methods comprise mechanical agitation of a suitable mixture containing the membrane-forming substance and water or an aqueous solution. Filtration through suitable membranes is preferred in order to form a substantially uniform liposome size.

The amphiphilic substances generally known in prior art to form liposome membranes can be employed in the context of the invention as long as they are pharmaceutically acceptable for the intended application. Presently, liposome-forming systems comprising lecithin are preferred. Such systems can comprise hydrogenated soy bean lecithin besides cholesterol and disodium succinatehexahydrate. It is presently specifically preferred to use hydrogenated soy bean lecithin as the sole membrane-forming agent. Commercially available products such as Phospholipon® 90 H (Aventis, Germany) or Lipoid S100-3 (Lipoid GmbH, Germany) are also preferred.

As can be taken from the review of Lauer A.C. et al. 1995 (*vide supra*) phospholipid-based liposomes may also be generally used for production of liposomes that transport their cargo through the skin. According to this review, the use of non-ionic liposomes, which can be formed with phosphatidylcholin, is also an option. Other components that may be used for the formation of micelles are also known to the person skilled in the art and may be used for the production of compositions according to the invention.

The average size of the particulate carrier in particular liposomes according to this invention can vary and varies preferably from about 1 nm to about 300 nm. Liposomes or particulate carriers having diameters in the range of about 50 nm and 200 nm are most preferred. Liposomes having diameters in the range of about 50 nm and 200 nm are most preferred.

Where alternative particulate carriers are used, they are generally prepared as known in the art. Thus, microspheres which are used to deliver a very wide range of therapeutic or cosmetic agents, are made as described for example in WO 95/15118.

Nanoparticles may in some cases be used, provided that they can be loaded with a sufficient amount of opioid. They can be prepared according to the methods known in the art.

Methods using a pulse laser deposition (PLD) apparatus and a polymeric target to apply coatings to drug powders in a short non-aqueous process are also suitable for the formation of particulate preparations according to this invention.

A further suitable delivery system employs Large Porous Particles as disclosed by David A. Edwards et al. in "Large Porous Particles for Pulmonary Drug Delivery" (Science, 20. June 1997, Vol. 276, p 1868-1871).

The inventive formulations comprise a particulate carrier composition comprising at least one opioid and/or optionally other actives as described above, and optionally additional adjuvants.

Generally, the amount of active such as an opioid in an inventive formulation will be determined by the desired effect on the one hand and the carrying capacity of the carrier composition for the actives such as an opioid and the maximum concentration of the carrier composition in the Formulation on the other hand. Broadly speaking, the amount of opioid(s) in an inventive carrier composition can range in concentrations between the lower limit of effectiveness of the opioid(s) and the maximum loading of the opioid(s) in the respective carrier composition and at a maximum concentration of the carrier composition in the formulation. It is understood that the opioid(s) are present in the inventive compositions in a pharmaceutically sufficient amount.

The preferred formulation comprises for example from 5 to 20 % w/w opioid, or from 10 to 15 % w/w opioid, in particular about 10 % w/w opioid, based on the weight of the free base, and is for example administered in doses of 10 to 1000 µg, or 10 to 100 µg of the formulation.

The opioid is preferably administered in doses of 1/10 to 1/1000 of the respective required intravenous dose.

In particular hydromorphone formulations are administered in dose ranges of 0,2 x 10⁻³ mg - 100 mg or 0,4 x 10⁻³ mg - 50 mg or 2 x 10⁻³ mg - 10mg or 0,2 x 10⁻³ mg - 0,19 mg hydromorphone.

In particular morphine formations are administered in dose ranges of 1 x 10⁻³ mg - 400 mg or 2 x 10⁻³ mg - 200 mg or 1 x 10⁻² mg - 40 mg or 1 x 10⁻² mg - 0,9 mg morphine.

In particular buprenorphine formations are administered in dose ranges of 0,003 x 10⁻³ mg - 0,3 mg or 0,006 x 10⁻³ mg - 0,15 mg or 0,03 x 10⁻³ mg - 0,03 mg or 0,003 x 10⁻³ - 0,0029 mg buprenorphine.

In particular fentanyl formations are administered in dose ranges of 0,005 x 10⁻³ mg - 0,5 mg or 0,01 x 10⁻³ mg - 0,25 mg or 0,05 x 10⁻³ mg - 0,05 mg or 0,005 x 10⁻³ mg - 0,0049 mg fentanyl.

In particular levomethadon administered in dose ranges of 0,025 x 10⁻³ mg - 2,5 mg or 0,05 x 10⁻³ mg - 1,25 mg or 0,025 x 10⁻² mg - 0,25 mg or 0,025 x 10⁻³ mg - 0,024 mg levomethadon.

In particular oxycodon administered in dose ranges of 1 x 10⁻³ mg - 200 mg or 2 x 10⁻³ mg - 100 mg or I x 10⁻² mg - 20 mg or 1 x 10⁻³ mg - 0,9 mg oxycodon.

In particular sulfentanyl administered in dose ranges of 0,0005 x 10⁻³ mg - 0,5 mg or 0,001 x 10⁻³ mg - 0,25 mg or 0,0005 x 10⁻² mg - 0,05 mg or 0,0005 x 10⁻³ mg - 0,00049 mg sulfentanyl.

In particular piritramid administered in doses of ranges of 0,1 x 10⁻³ mg - 80 mg or 0,02 x 10⁻³ mg - 40 mg or 0,1 x 10⁻² mg - 8 mg or 0,1 x 10⁻³ mg - 0,09 mg piritramid.

One preferred method for producing the invention's liposomal opioid composition can generally be described as follows:

The lipid membrane-forming components, e. g. lecithin, are dissolved in a suitable solvent such as chloroform or a 2: 1 mixture of methanol and chloroform and are filtered under sterile conditions. A lipid film is then produced on a sterile high surface substrate, such as glass beads, by controlled evaporation of the solvent. In some cases, it can be quite sufficient to form the film on the inner surface of the vessel used in evaporating the solvent without using a specific substrate to increase the surface.

An aqueous system is prepared from electrolyte components and the opioid(s) and/or other actives to be incorporated in the liposome composition. Such an aqueous system can e. g. comprise 10 mmol/l sodium hydrogen phosphate and 0.9 % sodium chloride, at pH 7.4; the aqueous system will further comprise the desired amount of actives/opioid(s). Often, the aqueous system will comprise an excess amount of the opioid(s) and/or other actives.

The liposomes are generally formed by agitating said aqueous system in the presence of said film formed by the lipid components. At this stage, further additives can be added to improve liposome formation; e. g. sodium cholate. Liposome formation is preferably influenced by mechanical action such as pressure filtration through e. g. polycarbonate membranes, or centrifuging. Generally, the raw liposome dispersion will be washed, e. g. with electrolyte solution as used in preparing the above-described solution of the active agent.

When liposomes with the required size distribution have been obtained and washed, they can be freeze dried and/or redispersed in a suitable carrier liquid at a suitable concentration to provide a liquid sprayable formulation.

The inventive formulations can also contain other customary agents, including adjuvants and additives, antioxidants, conserving agents or consistency-forming agents such as viscosity-adjusting additives, emulgators, etc. The person skilled in the art will select these adjuvants and additives in such a way that the ability of compositions substantially consisting of particulate carriers such as liposomes comprising the opioid(s), to comply with the intended application. Additives that mediate or enhance penetration of the liposomes into the skin may also be part of the inventive preparation. Such additives comprise e.g. DMSO or compounds selected from the group of fatty acids, fatty acid esters and fatty alcohols. Preferred formulations are in the form of a liquid sprayable formulation. Alternatively, the formulation may contain a dry particulate composition. Preferably, the formulation is administered as aerosol or spray formulation. Most preferred is an aerosol which is e.g. provided by a nebulizer device. Such devices usually contain a pressurized mixture of the formulation, liquid or dry, and a propellant. By actuation of the device a predetermined amount of a mixture is released from the device. Due to the sudden pressure decrease the propellant evaporates and the formulation is finely distributed/nebulized. Such nebulized formulations are e.g. capable of traveling deeply into the nasal passage and reaching the olefactory mucosa in the upper part of the nasal passage.

As already mentioned above, a particularly preferred embodiment of the invention is a formulation comprising a liposome composition comprising at least one opioid. Such compositions comprise Phospholipon® 90 H (Aventis, Germany) as liposome-forming material. Phospholipon® 90 H is a 90% hydrogenated phosphatidylcholin preparation which is more storage-stable than unhydrogenated phosphatidylcholin preparations. Citric acid and Na₂(HPO₄), which are used as buffering agents, advantageously influence the stability of the compositions.

### Method of Application/_{A}pplication Device

For an effective transport to the CNS, there should be only a short distance between the place of application (respective part of the surface skin or mucous membrane at the site of the target nerve tissue) and the target nerve tissue that will transport the opioid(s) directly to the brain or to the spinal cord. Suitable cerebral nerves are those that run close to the body surface. In humans the nervus olfactorius, nervus opticus and nervus accusticus are suitable. For the present invention the nervus olefactorius is preferred.

The inventive liposomal opioid formulation is applied to the skin or mucous membrane located at the site of the respective target nerve tissue, preferably the olefactory mucosa. The liposomal carriers loaded with the opioid(s) diffuse through the respective cutaneous barriers/membranes to the target nerves.

The cutanous/membrane barriers vary with the target nerve tissue and the respective place of application on the surface skin or mucous membrane, however, preferred is the olefactory mucosa.
Fig. 1 shows the location of the nervus olefactory in the human head.

For the delivery to the olfactory mucose according to one embodiment of the invention, a nebulizer device is used.

In particular, usefull are also devices that exploits the body's natural reflex that isolates the nasal circuit from the lungs during exhalation against a resistance. As the patient breathes into the device, the exhaled air actuates the delivery device, the airflow created by exhalation then carries the spray doplets into the nose and into the posterior nasal passage beyond the nasal valve, allowing deposition of active substance in the region of the olefactory bulb. At the same time the process of breathing-out automatically closes the nasal cavity, removing the risk of distribution to the lungs.

### Embodiment Examples

### Embodiment Example I

In a 1000 ml glass flask, provided with glass beads for increased surface, 51.9 mg cholesterol and 213 mg hydrogenated soy bean lecithin are dissolved in a sufficient amount of a mixture of methanol and chloroform in a 2:1 ratio. The solvent is then evaporated under vacuum until a film is formed on the inner surface of the flask and on the glass beads.

265 mg fentanyl citrate are separately dissolved/dispersed in 12 ml water.

In a fourth vessel, 900 mg saccharose and 57 mg disodium succinate are dissolved in 12 ml water.

The fentanyl citrate solution is then added to the lipid film in the flask and the mixture is shaken until the film is dissolved. The resulting raw liposome formulation is then subjected to a high pressure filtering step through a polycarbonate membrane to produce small, unilamellar liposomes with high amounts of encapsulated fentanyl citrate and a size form 50 to 200 nm. The high pressure filtering step can alternatively be employed after any subsequent washing step or instead high pressure homogenization or other prior art methods known to provide small uniform sized liposomes can be employed.

The liposome composition is then separated from the hydrated lipids in the flask. The product is centrifuged and the supernatant liquid is discarded. The saccharose solution is added ad 12 ml and the product is again centrifuged. Afterwards the supernatant liquid is again discarded. At this stage, a further washing step, using the saccharose solution or the sodium chloride buffer solution could be carried out.

For storage, the liposome composition can be freeze dried according to standard procedures.

### Embodiment Example II

In a 2000 ml flask provided with glass beads to increase surface, 173 mg hydrogenated soy bean lecithin and 90 mg disodium succinate are dissolved in approximately 60 ml of a methanol/chloroform mix in a 2:1 ratio. The solvent is removed under vacuum until a film is formed.

Again in a separate vessel, 8.77 g sodium chloride and 1.78 g Na₂HPO₄·2H₂O were dissolved in 400 ml water. Further water was added up to a total volume of 980 ml, and then, approximately 12 ml 1N hydrochloric acid were added to adjust pH to 7.4. This solution was then topped up with water to exactly 1000 ml.

173 g hydromorphone hydrochloride are dissolved/dispersed in 40 ml of the sodium chloride buffer solution and are added to the lipid film in the flask. The flask is then shaken until the film is dissolved and liposomes are formed.

As in example I, the resulting raw liposome formulation is then subject to a high pressure filtering step through a polycarbonate membrane to form unilamellar liposomes with a size from 50 to 200 nm . The high pressure filtering step can alternatively be employed after any subsequent washing step or instead high pressure homogenization or other prior art methods known to provide small uniform sized liposomes can be employed.

The product is centrifuged and the supernatant liquid is discarded.

To the thus produced liposome pellet, further 40 ml saccharose solution as described in Example I is added, and the centrifuging step is repeated. The supernatant is again discarded. At this stage, the washing step could be repeated where necessary.

For storage, the liposome composition can be freeze dried according to standard procedures.

From the liposome compositions of Examples I and II, the following formulations are made as described in subsequent Embodiment Example III.

### Embodiment Example III

The liposome compositions of examples I and II are mixed with a liquid carriers selected from the group of water, aqueous buffer solvents, mixtures of water and aqueous buffer solutions with polar organic solvents as e.g. ethanol to form formulations with different concentrations of fentanyl. Preferably formulations with 10 % w/w fentanyl or hydromorphone (calculated on the basis of fentanyl or hydromorphone free base) are formed. The resulting formulations preferably exhibit a viscosity which is suitable for spray applications and more preferred a viscosity substantially like water.

The formulation can be administered in 20 µg doses comprising an amount of fentanyl citrate or hydromorphone hydrochloride corresponding to 2 µg fentanyl or hydromorphone free base.

The examples provided above are not meant to be exclusive. Many other variations of the present invention would be obvious to those skilled in the art, and are contemplated to be within the scope of the appended claims.

## Claims

1. Formulation comprising at least one opioid substantially comprised in a particulate carrier composition wherein said carrier composition is capable of delivering the at least one opioid to cerebrospinal nerves or directly to the spinal cord upon application of said formulation to a mammal's surface skin or mucous membrane at the site of said cerebral nerves or the spinal cord, optionally across the respective cutaneous barriers between said cerebral nerves or the spinal cord and the surface of said skin or mucous membrane.

2. Formulation according to claim 1, wherein the cerebrospinal nerve is the nervus olefactorius, and the mucous membrane is the olefactory mucosa.

3. Formulation according to claim 1 or 2, wherein the size of the particulate carrier varies from 1 nm to 300 nm.

4. Formulation according to claim 1, wherein the size of the particulate carrier varies from 50 nm to 200 nm.

5. Formulation according to any preceding claim, wherein the at least one opioid is selected from the group of morphine, hydromorphone, buprenorphine, fentanyl, afentanyl, and sulfentanyl, tevomethadone, oxycodone and piritramide or pharmaceutically acceptable salts thereof.

6. Formulation according to any preceding claim, wherein the composition comprises a combination of an opioid agonist and an opioid antagonist.

7. Formulation according to any preceding claim, wherein the composition further comprises an active selected from the group of antidepressants.

8. Formulation according to any preceding claim wherein the carrier composition is a liposomol composition.

9. Formulation according to any preceding claim wherein the formulation is liquid and the carrier composition is a liposomal composition.

10. Formulation according to claim 9 comprising form 5 to 25 % w/w of the opioid, preferably from 10 to 20 % w/w of the opioid and most preferred about 10 % w/w of the opioid.

11. Formulation according to any preceding claim which is formulated as aerosol formulation.

12. Formulation according to claim 11 I which comprises a propellant.

13. Unit dose of 20 µg of a formulation according to any preceding claim.

14. Use of a formulation according to any preceding claim, for the manufacture of a medicament to treat pain in mammals.

15. Use according to claim 14, wherein the mammal is a human.

16. Use according to claim 14 or 15 wherein the medicament is used in the treatment of neuropathic pain.

17. Use according to claim 16, wherein the opioid substantially comprised in a particulate carrier composition is in the form of a formulation according to any preceding claim.

18. Use according to claims 14 to 17, wherein the formulation is administered to the olefactory mucosa.

19. A method of treating a mammal with an opioid analgesic, by applying to said mammal an opioid formulation as defined in claims 1 to 12,
wherein said opioid formulation is applied to said mammal's olefactory mucosa.
